# EUROPEAN PATENT APPLICATION

(11) **EP 2 623 094 A1**
(43) Date of publication of application: **07.08.2013**
(21) Application number: 12153582.7
(22) Date of filing: 02.02.2012
(51) Int. Cl.: A61K 8/97, A61Q 19/08, A61P 17/00

(54) **Use of an edelweiss extract**

(71) Applicant: DSM IP Assets B.V., 6411 TE Heerlen (NL)
(72) Inventor: The designation of the inventor has not yet been filed
(74) Representative: Schwander, Kuno

(57) **Abstract**

The present invention relates to a novel topical use of an edelweiss extract as a skin active agent for protection, strengthening, improvement and/or regeneration of skin barrier function as well as for prevention against skin barrier damages.

## Description

The present invention relates to a novel topical use of an edelweiss extract as a skin active agent for protection, strengthening, improvement and/or regeneration of skin barrier function as well as for prevention against skin barrier damages.

More particularly, the present invention relates to an edelweiss extract for use in topical compositions as a skin active agent for protection, strengthening, improvement and/or regeneration of skin barrier function as well as for prevention against skin barrier damages which comprises topically administering an effective amount an edelweiss extract to the appropriate skin area of a person in need of such agent, or treatment.

There is a growing demand in the industry, and a growing market need for cosmetic skin care products that improve or regenerate the natural skin barrier functionality, and even prevent the degradation of the skin barrier.

The skin is the single largest organ of the body. The skin, when healthy, protects the individual from chemical, physical, and biological hazards via specific skin barrier mechanisms. Skin weighs about 10% of our total body weight and is approximately one eighth of an inch thick. The skin is made up of two layers, the epidermis (outer layer) and the dermis (inner layer).

The epidermis is a highly specialized epithelium performing multiple protective and vital functions. In particular it prevents water loss, excludes environmental toxins, resists mechanical stress and UV radiation and participates in immune defence. In fact the epidermis maintains several types of barriers representing multiple aspects of protection and defence either outside-in, but also inside-out. Therefore a well balanced, stable and strong skin barrier is essential not only to prevent disordered conditions or even diseases but also for well being, a positive skin sensation, a healthy and attractive appearance.

Thus, there is an ongoing need for highly efficient cosmetic skin care products having a skin protective effect thereby, preventing skin damages when exposed to environmental stresses, but also improving and/or regenerating the skin barrier and overcoming the drawbacks of the products of the prior art. Typically the products of the prior art address a single component of the very complex skin barrier functionality. e.g.: antioxidants and UV filters are used as protective agents and address damages caused by free radicals or prevent their formation. Oils and lipids as well as products like allantoin, panthenol, aloe vera are used as regenerative agents. UV filters specifically give a physical protection from irradiation (prevention of radical formation) but they do not stimulate the endogenous protective capacity of the skin against UV rays nor do they improve the skin barrier itself. Similarly, regenerative agents as mentioned above do have a specific barrier protection effect or an antioxidant capacity, however they do not have multifunctional barrier protection capacities.

Surprisingly, the inventors of the present invention found that an edelweiss extracts, acts very efficiently in modulating key skin barrier biomarkers, thereby resulting in a multifunctional and multidimensional synergistic endogenous barrier activation simultaneously acting at the levels of keratinocyte cell differentiation (cellular barrier), lipid synthesis (lipid barrier), and defensin activation (antimicrobial barrier).

Therefore, the first object of the present invention is the use of a topical composition comprising an edelweiss extract for prevention of skin damages, and/or regeneration of the skin barrier function, and/or for strengthening of the skin barrier, and/or improvement of the natural skin protection functionality. In a preferred embodiment, the improvement of the natural skin protection functionality is characterized by induction of cellular differentiation markers, induction of genes contributing to barrier lipid synthesis, and induction of endogenous anti-microbial proteins.

In another embodiment, the improvement of the natural skin protection functionality is characterized by an improved skin appearance and skin moisturization.

According to the present invention, cell differentiation markers are selected from keratin 1, keratin 10, small proline rich protein 1B, and involucrine. Barrier lipid synthesis genes are selected from fatty acid binding protein 5, glucosylceramidease, acid sphygomyelin phosphodiesterase 1, and cytosolic sulfotransferase 2B. Antimicrobial barrier marker is defensin beta 4.

The edelweiss extract used in the present invention is derived from the aerial parts (flowers) of *Leontopodium alpinum* Cass. *Leontopodium alpinum* Cass. (Edelweiss) is one of the most famous plants in the European Alps and grows in high altitude. Typically, such an extract is rich in phenolic acids, Leontopodic acid (a highly substituted hexaric acid derivative), which is well known to have powerful antioxidant properties. It is also rich in other phenolic acids (chlorogenic acid), flavonoids (luteolin-4'-O-glucoside, apigenin-7-O-glucoside, luteolin), and tannin. In a preferred embodiment, the extract is as described in (WO2001/087256) and is commercialized under the brand name of ALPAFLOR^{®} EDELWEISS by DSM Nutritional Products. Cosmetics formulations aiming at sun protection, anti-aging, and anti-oxidative treatments are being formulated using Edelweiss extracts.

Typically, the edelweiss extract is prepared as follows: Aerial parts of Leontopodium alpinum are dried under hot air flow, followed by milling. The dried plants are then extracted with an ethanol/water solution. The ethanol is then removed by vacuum distillation, and the concentrate is diluted with glycerin, or a glycerin/ethanol mixture.

Preferably, the topical composition as used in the present invention is a cosmetic composition or a pharmaceutical composition. Most preferably, it is a cosmetic composition for skin care. In a preferred embodiment, the topical composition comprises between 0.001 and 10 weight-% edelweiss extract. More preferably, the topical composition comprises between 0.01 and 10 weight-%, even more preferably, it comprises between 0.1 and 5 weight-%, most preferably between 0.5 and 3 weight-% edelweiss extract.

According to the present invention, the edelweiss extract can be used as such or in an encapsulated form, for example in a liposomal form. Liposomes are preferably formed with lecithins with or without addition of sterols or phytosterols. The encapsulation of the active ingredients can be alone or together with other active ingredients. Other embodiments include solid or semisolid capsules aiming to protect the edelweiss extract from degradation or for controlled delivery. Suitable encapsulation technologies are for example described in WO 0180823, WO 9903450, WO 9317784 or in Fragrance Journal (2001), 29(2), 83-90.

Preferred topical cosmetic or pharmaceutical compositions according to the invention are skin (face and body) care preparations, decorative preparations, light protection preparations and functional preparations.

Examples of skin care preparations are, in particular, face creams, body oils, body lotions, body gels, treatment creams, skin protection ointments, shaving preparations, such as shaving foams or gels, moisturizing gels, moisturizing sprays, revitalizing body sprays, cellulite gels, face and/or body moisturizers, facial and/or body cleansers, and face masks. Most preferred are face care products.

Preferred topical cosmetic or pharmaceutical compositions according to the invention are skin care preparations, or functional preparations.

Examples of decorative preparations are, in particular, lipsticks, eye shadows, mascaras, dry and moist make-up formulations, rouges, powders, and/or suntan lotions.

Examples of functional preparations are cosmetic compositions containing further active ingredients such as hormones, vitamins, vegetable and/or fruit extracts, anti-ageing ingredients, and/or antimicrobial (antibacterial or antifungal) ingredients without being limited thereto.

Cosmetic compositions for use in accordance with the invention can be in the form of a liquid, lotion, a thickened lotion, a gel, a cream, a milk, an ointment, a paste, a powder, a make-up, or a solid tube stick and can be optionally be packaged as an aerosol and can be provided in the form of a mousse such as a aerosol mousse, a foam or a spray foams, sprays, sticks, a gel, a plaster, a powder, a cleanser, a soap or aerosols or wipes. Preferred topical compositions comprise a cream, an emulsion, a gel, an ointment, a lotion a tincture, a spray, a mousse, a cleansing composition or foam.

In another embodiment, the topical composition for use according to the present invention is characterized in that it comprises at least one UV screening agent, and/or a moisturizer, and/or an anti-aging agent, and/or a skin tone agent, and a conventional carrier.

Preferably, the topical composition for use according to the present invention is characterized in that it comprises at least one UV screening agent, and a conventional carrier.

The additional UV-screening agents are advantageously selected from IR, UV-A, UV-B, UV-C and/ or broadband filters. Examples of UV-B or broad spectrum screening agents, i.e. substances having absorption maximums between about 290 nm and 340 nm may be organic or inorganic compounds. Organic UV-B or broadband screening agents are e.g. acrylates such as 2-ethylhexyl 2-cyano-3,3-diphenylacrylate (octocrylene, PARSOL^{®} 340), ethyl 2-cyano-3,3-diphenylacrylate and the like; camphor derivatives such as 4-methyl benzylidene camphor (PARSOL^{®} 5000), 3-benzylidene camphor, camphor benzalkonium methosulfate, polyacrylamidomethyl benzylidene camphor, sulfo benzylidene camphor, sulphomethyl benzylidene camphor, therephthalidene dicamphor sulfonic acid and the like; Cinnamate derivatives such as ethylhexyl methoxycinnamate (PARSOL^{®} MCX), ethoxyethyl methoxycinnamate, diethanolamine methoxycinnamate (PARSOL^{®} Hydro), isoamyl methoxycinnamate and the like as well as cinnamic acid derivatives bond to siloxanes; p-aminobenzoic acid derivatives, such as p-aminobenzoic acid, 2-ethylhexyl p-dimethylaminobenzoate, N-oxypropylenated ethyl p-aminobenzoate, glyceryl p-aminobenzoate; benzophenones such as benzophenone-3, benzophenone-4, 2,2',4,4'-tetrahydroxy-benzophenone, 2,2'-dihydroxy-4,4'-dimethoxybenzophenone and the like; esters of benzalmalonic acid such as di-(2-ethylhexyl) 4-methoxybenzalmalonate; esters of 2-(4-ethoxy-anilinomethylene)propandioic acid such as 2-(4-ethoxy anilinomethylene) propandioic acid diethyl ester as described in the European Patent Publication EP 0895 776; organosiloxane compounds containing benzmalonate groups as described in the European Patent Publications EP 0358584 B1, EP 0538431 B1 and EP 0709080 A1 such as PARSOL^{®} SLX; drometrizole trisiloxane (Mexoryl XL); imidazole derivatives such as e.g. 2-phenyl benzimidazole sulfonic acid and its salts (PARSOL^{®}HS). Salts of 2-phenyl benzimidazole sulfonic acid are e.g. alkali salts such as sodium- or potassium salts, ammonium salts, morpholine salts, salts of primary, sec. and tert. amines like monoethanolamine salts, diethanolamine salts and the like; salicylate derivatives such as isopropylbenzyl salicylate, benzyl salicylate, butyl salicylate, ethylhexyl salicylate (PARSOL^{®} EHS, Neo Heliopan OS), isooctyl salicylate or homomenthyl salicylate (homosalate, PARSOL^{®} HMS, Neo Heliopan HMS) and the like; triazine derivatives such as ethylhexyl triazone (Uvinul T-150), diethylhexyl butamido triazone (Uvasorb HEB) and the like. Encapsulated UV-filters such as encapsulated ethylhexyl methoxycinnamate (Eusolex UV-pearls) or microcapsules loaded with UV-filters as e.g. dislosed in EP 1471995 and the like; Examples of broad spectrum or UV A screening agents i.e. substances having absorption maximums between about 320 nm and 400 nm may be organic or inorganic compounds. Organic broad spectrum or UV A screening agents include e.g. dibenzoylmethane derivatives such as 4-tert.-butyl-4'-methoxydibenzoyl-methane (PARSOL^{®} 1789), dimethoxydibenzoylmethane, isopropyldibenzoylmethane and the like; benzotriazole derivatives such as 2,2'-methylene-bis-(6-(2H-benzotriazole-2-yl)-4-(1,1,3,3,-tetramethylbutyl)-phenol (Tinosorb M) and the like; bis-ethylhexyloxyphenol methoxyphenyl triazine (Tinosorb S) and the like; phenylene-1,4-bis-benzimidazolsulfonic acids or salts such as 2,2-(1,4-phenylene)bis-(1H-benzimidazol-4,6-disulfonic acid) (Neoheliopan AP); amino substituted hydroxybenzophenones such as 2-(4-Diethylamino-2-hydroxy-benzoyl)-benzoic acid hexylester (Uvinul A plus) as described in the European Patent Publication EP 1046391; Ionic UV-A filters as described in the International Patent Publication WO2005080341 A1; As dibenzoylmethane derivatives have limited photostability it may be desirable to photostabilize these UV-A screening agents. Thus, the term "conventional UV-A screening agent" also refers to dibenzoylmethane derivatives such as e.g. PARSOL^{®} 1789 stabilized by, e.g. 3,3-Diphenylacrylate derivatives as described in the European Patent Publications EP 0 514 491 B1 and EP 0 780 119 A1; Benzylidene camphor derivatives as described in the US Patent No. 5,605,680; Organosiloxanes containing benzmalonate groups as described in the European Patent Publications EP 0358584 B1, EP 0538431 B1 and EP 0709080 A1.

A good overview of UV-A- and UV-B screening agents which can be added to the compositions of the present invention can also be found in DE-A 103 27 432. All UV-filter compounds disclosed in this document are also useful as components for the compositions of the present invention and are included herein by reference.

A safe and effective amount of the UV-screening agent is used, typically from about 1 wt.-% to about 20 wt.-%, more typically from about 2 wt.-% to about 10 wt.-%.

Other suitable UV-screening agents which may be incorporated into the topical cosmetic or pharmaceutical compositions of the present invention are inorganic pigments such as microparticulated metal oxides (e.g. PARSOL^{®} TX). Examples of such compounds include e.g. titanium dioxide having an average primary particle size of from about 15 nm to about 100 nm, zinc oxide having an average primary particle size of from about 15 nm to about 150 nm, zirconium oxide having an average primary particle size of from about 15 nm to about 150 nm, iron oxide having an average primary particle size of from about 15 nm to about 500 nm, and mixtures thereof. The metal oxide particles may also be coated by metal oxides such as e.g. aluminum or zirconium oxides or by organic coatings such as e.g. polyols, methicone, aluminum stearate, alkyl silane. Such coatings are well known in the art. When used herein, the inorganic sunscreens are present in the amount of from about 0.1 wt.-% to about 20 wt.-%, preferably from about 0.5 wt.-% to about 10 wt.-%, more preferably from about 1wt.-% to about 5 wt.-%.

Conventional carriers comprise excipients or diluents conventionally used in topical compositions. If nothing else is stated, the excipients, additives, diluents, etc. mentioned in the following are suitable for both pharmaceutical and cosmetic compositions. The necessary amounts of the cosmetic and dermatological adjuvants and additives can, based on the desired product, easily be determined by the skilled person.

Regarding the kind of the topical cosmetic and pharmaceutical composition and the preparation of the topical cosmetic and pharmaceutical preparations as well as for further suitable additives, it can be referred to the pertinent literature, e.g. to Novak G.A., Die kosmetischen Präparate - Band 2, Die kosmetischen Präparate - Rezeptur, Rohstoffe, wissenschaftliche Grundlagen (Verlag für Chem. Industrie H. Ziolkowski KG, Augsburg).

Preferably, the topical cosmetic or pharmaceutical compositions used in the present invention are in the form of a suspension or dispersion in solvents or fatty substances, or alternatively in the form of an emulsion or micro emulsion (in particular of O/W or W/O type, O/W/O or W/O/W-type), PET-emulsions, multiple emulsions, bickering emulsions, hydrogels, alcoholic gels, lipogels, one or multiphase solutions or a vesicular dispersion and other usual compositions, which can also be applied by pens, as masks or as sprays. The emulsions can also contain anionic, nonionic, cationic or amphoteric surfactant(s).

The topical cosmetic or pharmaceutical compositions used in the present invention can also contain usual cosmetic or pharmaceutical adjuvants and additives, such as preservatives/ antioxidants, fatty substances/ oils, water, organic solvents, silicones, thickeners, softeners, emulsifiers, sunscreens, antifoaming agents, moisturizers, fragrances, surfactants, fillers, sequestering agents, anionic, cationic, nonionic or amphoteric polymers or mixtures thereof, propellants, acidifying or basifying agents, dyes, colorants, pigments or nanopigments, e.g. those suited for providing a photoprotective effect by physically blocking out ultraviolet radiation, or any other ingredients usually formulated into topical cosmetic or pharmaceutical compositions. The necessary amounts of the cosmetic and dermatological adjuvants and additives can, based on the desired product, easily be chosen by a skilled artisan in this field and will be illustrated in the examples, without being limited hereto. The usual cosmetic adjuvants and additives such as emulsifiers, thickeners, surface active ingredients and film formers can show synergistic which can be determined by the expert in the field with normal trials, or with the usual considerations regarding the formulation of topical cosmetic or pharmaceutical composition.

Typically topical cosmetic or pharmaceutical compositions also contain surface active ingredients like emulsifiers, solubilizers and the like. An emulsifier enables two or more immiscible components to be combined homogeneously. Moreover, the emulsifier acts to stabilize the composition. Solubilizers that may be used in the present invention include but are not restricted to PEG/PPG-18/18 Dimethicone, PEG-40 Hydrogenated Castor Oil, PEG-20 Stearate, PEG-30 Glyceryl Stearate, and PEG-7 Glyceryl Cocoate. Emulsifiers that may be used in the present invention in order to form O/W, W/O, O/W/O or W/O/W emulsions/ microemulsions include sorbitan oleate, sorbitan sesquioleate, sorbitan isostearate, sorbitan trioleate, polyglyceryl-3-diisostearate, polyglycerol esters of oleic/isostearic acid, polyglyceryl-6 hexaricinolate, polyglyceryl-4-oleate, polyglyceryl-4- oleate/PEG-8 propylene glycol cocoate, oleamide DEA, TEA myristate, TEA stearate, magnesium stearate, sodium stearate, potassium laurate, potassium ricinoleate, sodium cocoate, sodium tallowate, potassium castorate, sodium oleate, and mixtures thereof. Furthermore, one or more synthetic polymers may be used as an emulsifier. For example, PVP eicosene copolymer, acrylates/C₁₀₋₃₀ alkyl acrylate crosspolymer, acrylates/steareth-20 methacrylate copolymer, PEG-22/dodecyl glycol copolymer, PEG-45/dodecyl glycol copolymer, and mixtures thereof. The preferred emulsifiers are PVP Eicosene copolymer, acrylates/C₁₀₋₃₀-alkyl acrylate crosspolymer, PEG-20 sorbitan isostearate, sorbitan isostearate, and mixtures thereof. The one or more emulsifiers are present in a total amount about 0.01 wt.% to about 20 wt.% of the total weight of the topical cosmetic or pharmaceutical topical composition for use in the present invention. Preferably, about 0.1 wt.% to about 10 wt.% of emulsifiers is used.

The lipid phase of the topical cosmetic or pharmaceutical compositions can advantageously be chosen from: mineral oils and mineral waxes; oils such as triglycerides of caprinic acid or caprylic acid and castor oil; oils or waxes and other natural or synthetic oils, in a preferred embodiment esters of fatty acids with alcohols e.g. isopropanol, propylene glycol, glycerin or esters of fatty alcohols with carboxylic acids or fatty acids; alkylbenzoates; and/or silicone oils such as dimethylpolysiloxane, diethylpolysiloxane, diphenylpolysiloxane, cyclomethicones and mixtures thereof.

Exemplary fatty substances which can be incorporated in the oil phase of the emulsion, microemulsion, oleo gel, hydrodispersion or lipodispersion of the topical cosmetic or pharmaceutical composition of the present invention are advantageously chosen from esters of saturated and/or unsaturated, linear or branched alkyl carboxylic acids with 3 to 30 carbon atoms, and saturated and/or unsaturated, linear and/or branched alcohols with 3 to 30 carbon atoms as well as esters of aromatic carboxylic acids and of saturated and/or unsaturated, linear or branched alcohols of 3-30 carbon atoms. Such esters can advantageously be selected from octylpalmitate, octylcocoate, octylisostearate, octyldodecylmyristate, cetearylisononanoate, isopropylmyristate, isopropylpalmitate, isopropylstearate, isopropyloleate, n-butylstearate, n-hexyllaureate, n-decyloleate, isooctylstearate, isononylstearate, isononylisononanoate, 2- ethyl hexylpalmitate, 2-ethylhexyllaurate, 2-hexyldecylstearate, 2-octyldodecylpalmitate, stearylheptanoate, oleyloleate, oleylerucate, erucyloleate, erucylerucate, tridecylstearate, tridecyltrimellitate, as well as synthetic, half-synthetic or natural mixtures of such esters e.g. jojoba oil.

Other fatty components suitable for use in the topical cosmetic or pharmaceutical compositions for use according to the present invention include polar oils such as lecithins and fatty acid triglycerides, namely triglycerol esters of saturated and/or unsaturated, straight or branched carboxylic acid with 8 to 24 carbon atoms, preferably of 12 to 18 carbon-atoms whereas the fatty acid triglycerides are preferably chosen from synthetic, half synthetic or natural oils (e.g. cocoglyceride, olive oil, sun flower oil, soybean oil, peanut oil, rape seed oil, sweet almond oil, palm oil, coconut oil, castor oil, hydrogenated castor oil, wheat oil, grape seed oil, macadamia nut oil and others); apolar oils such as linear and/ or branched hydrocarbons and waxes e.g. mineral oils, vaseline (petrolatum); paraffins, squalane and squalene, polyolefins, hydrogenated polyisobutenes and isohexadecanes, favored polyolefins are polydecenes; dialkyl ethers such as dicaprylylether; linear or cyclic silicone oils such as preferably cyclomethicones (octamethylcyclotetrasiloxane; cetyldimethicone, hexamethylcyclotrisiloxane, polydimethylsiloxane, poly(methylphenylsiloxane) and mixtures thereof.

Other fatty components which can advantageously be incorporated in topical cosmetic or pharmaceutical compositions for use according to the present invention are isoeikosane; neopentylglycoldiheptanoate; propyleneglycoldicaprylate/ dicaprate; caprylic/ capric/diglycerylsuccinate; butyleneglycol caprylat/caprat; C₁₂₋₁₃-alkyllactate; di-C₁₂₋₁₃ alkyltartrate; triisostearin; dipentaerythrityl hexacaprylat/hexacaprate; propylene-glycolmonoisostearate; tricaprylin; dimethylisosorbid. Especially beneficial is the use of mixtures C₁₂₋₁₅-alkylbenzoate and 2-ethylhexylisostearate, mixtures C₁₂₋₁₅-alkylbenzoate and isotridecylisononanoate as well as mixtures of C₁₂₋₁₅-alkylbenzoate, 2-ethylhexylisostearate and isotridecylisononanoate.

The oily phase of the topical cosmetic or pharmaceutical compositions for use according to the present invention can also contain natural vegetable or animal waxes such as bee wax, china wax, bumblebee wax and other waxes of insects as well as Shea butter and cocoa butter.

A moisturizing agent may be incorporated into a topical cosmetic or pharmaceutical composition used according to the present invention to maintain hydration or rehydrate the skin. Moisturizers that prevent water from evaporating from the skin by providing a protective coating are called emollients. Additionally an emollient provides a softening or soothing effect on the skin surface and is generally considered safe for topical use. Preferred emollients include mineral oils, lanolin, petrolatum, capric/caprylic triglyceraldehydes, cholesterol, silicones such as dimeticone, cyclometicone, almond oil, jojoba oil, avocado oil, castor oil, sesame oil, sunflower oil, coconut oil and grape seed oil, cocoa butter, olive oil aloe extracts, fatty acids such as oleic and stearic, fatty alcohols such as cetyl and hexadecyl (ENJAY), diisopropyl adipate, hydroxybenzoate esters, benzoic acid esters of C₉₋₁₅-alcohols, isononyl iso-nonanoate, ethers such as polyoxypropylene butyl ethers and polyoxypropylene cetyl ethers, and C₁₂₋₁₅- alkyl benzoates, and mixtures thereof. The most preferred emollients are hydroxybenzoate esters, aloe vera, C₁₂₋₁₅-alkyl benzoates, and mixtures thereof. An emollient is present in an amount of about 1 wt.% to about 20 wt.% of the total weight of the topical cosmetic or pharmaceutical composition. The preferred amount of emollient is about 2 wt.% to about 15 wt.%, and most preferably about 4 wt.% to about 10 wt.%.

Moisturizers that bind water, thereby retaining it on the skin surface are called humectants. Suitable humectants can be incorporated into a topical cosmetic or pharmaceutical composition of the present invention such as glycerin, polypropylene glycol, polyethylene glycol, lactic acid, pyrrolidone carboxylic acid, urea, phospholipids, collagen, elastin, ceramides, lecithin sorbitol, PEG-4, and mixtures thereof. Additional suitable moisturizers are polymeric moisturizers of the family of water soluble and/ or swellable/ and/ or with water gelating polysaccharides such as hyaluronic acid, chitosan and/or a fucose rich polysaccharide which is e.g. available as Fucogel®1000 (CAS-Nr. 178463-23-5) by SOLABIA S. One or more humectants are optionally present at about 0.5 wt.% to about 8 wt.% in a topical cosmetic or pharmaceutical composition of the present invention, preferably about 1 wt.% to about 5 wt.%.

The aqueous phase of the preferred topical cosmetic or pharmaceutical compositions for use in the present invention can contain the usual cosmetic or pharmaceutical additives such as alcohols, especially lower alcohols, preferably ethanol and/ or isopropanol, low diols or polyols and their ethers, preferably propyleneglycol, glycerin, ethyleneglycol, ethyleneglycol monoethyl- or monobutylether, propyleneglycol monomethyl- or - monoethyl- or-monobutylether, diethyleneglycol monomethyl-or monoethylether and analogue products, polymers, foam stabilizers; electrolytes and especially one or more thickeners. However, preferably the topical cosmetic or pharmaceutical compositions for use in the present invention are free of ethanol, more preferably they are free of alcohols, and most preferably they are free of organic solvents, since such compounds can cause skin irritation.

Thickeners that may be used in topical cosmetic or pharmaceutical compositions for use in the present invention to assist in making the consistency of a product suitable include carbomer, siliciumdioxide, magnesium and/ or aluminium silicates, beeswax, stearic acid, stearyl alcohol polysaccharides and their derivatives such as xanthan gum, hydroxypropyl cellulose, polyacrylamides, acrylate crosspolymers preferably a carbomer, such as carbopole^{®} of type 980, 981, 1382, 2984, 5984 alone or mixtures thereof.

Suitable neutralizing agents which may be included in the topical cosmetic or pharmaceutical composition of the present invention to neutralize components such as e.g. an emulsifier or a foam builder/stabilizer include but are not limited to alkali hydroxides such as a sodium and potassium hydroxide; organic bases such as diethanolamine (DEA), triethanolamine (TEA), aminomethyl propanol, and mixtures thereof; amino acids such as arginine and lysine and any combination of any foregoing. The neutralizing agent can be present in an amount of about 0.01 wt.% to about 8 wt.% in the topical cosmetic or pharmaceutical composition of the present invention, preferably, 1 wt.% to about 5 wt.%.

The addition of electrolytes into the topical cosmetic or pharmaceutical composition for use according to the present invention may be necessary to change the behavior of a hydrophobic emulsifier. Thus, the emulsions/ microemulsions for use according to this invention may contain preferably electrolytes of one or several salts including anions such as chloride, sulfates, carbonate, borate and aluminate, without being limited thereto. Other suitable electrolytes can be on the basis of organic anions such as, but not limited to, lactate, acetate, benzoate, propionate, tartrate and citrate. As cations preferably ammonium, alkylammonium, alkali- or alkaline earth metals, magnesium-, iron- or zinc-ions are selected. Especially preferred salts are potassium and sodium chloride, magnesium sulfate, zinc sulfate and mixtures thereof. Electrolytes can be present in an amount of about 0.01 wt. % to about 8 wt. % in the topical cosmetic or pharmaceutical composition of the present invention.

It is also an object of the present invention to provide a topical pharmaceutical composition comprising an edelweiss extract for use in the prevention and/or treatment of human skin barrier abnormalities. Preferred skin barrier abnormalities prevented and/or treated by the edelweiss extract are selected from atopic dermatitis, allergic skin, psoriatic skin inflamed skin, abnormal skin re-epithelization, burned or sunburned skin, and wounded skin.

The use of the topical cosmetic or pharmaceutical compositions according to the present invention is preferably performed by application at least once per day, e.g. twice or triple times a day on the skin, preferably the face.

The present invention also relates to a method of regeneration of the skin barrier function and/or improvement of the natural skin protection mechanism which comprises topically administering an effective amount of an edelweiss extract to the appropriate skin area of a person in need of such treatment. Furthermore, it also relates to a method of preventing skin barrier abnormalities which comprises topically administering an effective amount of an edelweiss extract to the appropriate skin area of a person in need of such treatment.

The term 'an effective amount' refers to an amount necessary to obtain a physiological effect. The physiological effect may be achieved by one application dose or by repeated applications. The dosage administered may, of course, vary depending upon known factors, such as the frequency of treatment; and the effect desired and can be adjusted by a person skilled in the art.

The present invention also relates to a method as described above, wherein, from about 0.2 µg to about 200 µg of an edelweiss extract, is applied per square centimeter of skin per day.

The present invention also relates to a method as described above, wherein at least one additional UV screening agent and/or a moisturizer, and/or an anti-aging agent, and/or a skin tone agent is administered.

The usefulness of agents for regeneration of the skin barrier function and/or improvement of the skin barrier function can be determined by methods known in the art. The efficacy can be measured on in-vitro keratinocyte cell cultures by gene expression analysis or ELISA protein quantification or immuno-flurorescent staining for barrier markers involved in keratinocyte differentiation, barrier lipid synthesis and innate immunity in particular antimicrobial peptides. In-vivo the benefit can easily be shown by measuring a reduction of trans-epidermal water loss (TEWL) also after tape stripping, by reduction of skin roughness (Primos), reduction of skin lesions and a better skin appearance.

The following Examples are illustrative but not limitative of the invention.

### Examples

### Example 1: Assessment of the effect of an edelweiss extract on normal epidermal keratinocytes (NHEK)

1- Material and methods:
A) Test material and preparation: Edelweiss extract (ALPAFLOR^{®} EDELWEISS from DSM) at a concentration of 5 wt-% dry residue was prepared as a stock solution in 60% ethanol. The stock solution was diluted into the cell culture media to the final test concentration.
B) Biological model:
   Normal human epidermal keratinocytes (NHEK), reference K544 used at the 3^{rd} passage Culture conditions: 37°C, 5% CO₂
   Culture medium: Keratinocytes SFM supplemented with epidermal growth factor (EGF) 0.25ng/ml, Pituitary extract (PE) 25 µg/ml, Gentamycin 25 µg/ml
   Assay medium: Keratinocyte-SFM supplemented with Gentamycin 25 µg/ml.
C) Cytotoxicity preliminary assay (MTT):
   NHEK were seeded at 10'000 cells/well and incubated for 48 hours. Cytotoxicity was quantified by the MTT reduction assay and morphological observations were done with a microscope.
D) Keratinocyte culture treatment:
   Keratinocytes were seeded in culture medium and incubated for 24 hours, and then the culture medium was replaced by assay medium for 24 hours. Then the assay medium was replaced again with medium containing (or not for the control) the extract. Cells were then incubated for either 24 hours or 48 hours with the extract. Each experimental condition was performed in triplicates. At the end of the incubation time the supernatants were discarded, the cells were washed in phosphate buffered saline (PBS) solution and immediately frozen at -80°C.
E) Analysis of Gene Expression by Quantitative PCR technology as PCR array:
   The expression of a selection of genes was analysed using RT-qPCR method on mRNA extracted from the cell monolayers for each treatment. Before RNA extraction the replicas were pooled. The analysis of gene expression was then done in duplicates (n=2).
Reverse transcription: Total RNA was extracted from each sample using TriPure solution reagent according to the supplier's advices. The amount and quality of RNA were evaluated using a lab-on-a-chip Bioanalyzer (Agilent Technologies). Potential contaminant traces of genomic DNA were removed using the DNAfree system (Ambion). The amount of the RNA was evaluated using Nanovue (GE Healthcare). The reverse-transcription of mRNA was conducted in presence of oligo(dT) and Superscript II reverse-transcriptase (Invitrogen). Nanovue was also used for quantification of cDNA and DNA concentration adjustment.

Quantitative PCR: The PCRs (Polymerase Chain Reactions) were performed using the Light Cycler system (Roche Molecular Systems Inc.) according to the supplier's instructions. This system allows rapid and powerful PCRs after determining analysis conditions of the test primers. The reaction mix contains diluted DNA, primers forward and reverse, reagent mix containing taq DNA polymerase, SYBR Green I and MgCl2. The incorporation of fluorescence in amplified DNA was continuously measured during the PCR cycles. This resulted in a fluorescence intensity versus PCR cycle plot allowing the evaluation of a relative expression (RE) value for each gene. The value selected for RE calculations is the output point (Ct) of the fluorescence curve. For the considered gene the higher the cycle number the lower is the mRNA quantity. The RE value was expressed in arbitrary units (AU) according to the formula: (1/2number of cycles) x 106. For additional control we also quantified the expression level of three housekeeping genes namely Ribosomal protein S28, Glyceraldehyde-3-phosphate dehydrogenase and beta-Actin in both untreated control cells and treated cells. The mean relative expression level of the three housekeeping genes was set to 100% and the variation in expression level of individual three genes was within less than 100 ± 50%.

### 2- Results:

The edelweiss extract modulated the gene expression of following key barrier markers as summarized in Table 1:
   1. Differentiation markers like keratin 1 and keratin 10, transglutaminase 1, loricrin, small prolin rich protein 1B, and involucrin were upregulated. These proteins are involved in cornified envelope formation or "cellular barrier" formation and thus represent one part of the permeability barrier of the stratum corneum.
   2. Enzymes involved in barrier lipid synthesis like fatty acid binding protein 5, glucosylceramidase, acid sphingomyelin phosphodiesterase 1, and cytosolic sulfotransferase 2B were upregulated. These proteins play an important role in the setup of the lipid barrier which represents the other part of the permeability barrier.
   3. Antimicrobial peptides like defensin beta 4 were upregulated. These proteins support the endogenous antimicrobial barrier of the skin. 5

**Table 1: Gene expression results in normal human keratinocytes expressed as percentage of expression compared to control at two time points:**

| | | | 24 hours with Edelweiss Extract - 0.001% | 48 hours with Edelweiss Extract - 0.001% |
|---|---|---|---|---|
| | **Gene code** | **Gene name** | **% Control (Mean HK)** | **% Control (Mean HK)** |
| | | | 100 | 100 |
| **Housekeeping** | RPS28 | Ribosomal protein S28 | 126 | 125 |
| | GAPDH | Glyceraldehyde-3-phosphate dehydrogenase | 124 | 123 |
| | ACTB | Actin, beta | 75 | 77 |
| **Keratinocyte differentiation** | | | | |
| | CRNN | Cornulin | 142 | 241 |
| | FLG | Filaggrin | 823 | 819 |
| | IVL | Involucrin | 203 | 749 |
| | KRT1 | Keratin 1 | 4164 | 86028 |
| | KRT10 | Keratin 10 | 145 | 793 |
| | LOR | Loricrin | 467 | 310 |
| | SPRR1 A | Small proline-rich protein 1A | 188 | 776 |
| | SPRR1B | Small proline-rich protein 1 B (cornifin) | 376 | 1077 |
| | TGM1 | Transglutaminase 1 (K polypeptide epidermal type I, protein-glutamine-gamma-glutamyltransferase) | 381 | 304 |
| **Lipid synthesis** | FABP5 | Fatty acid binding protein 5 (psoriasis-associated) | 187 | 564 |
| | GBA | Glucosidase, beta; acid (includes glucosylceramidase) | 252 | 314 |
| | SMPD1 | Sphingomyelin phosphodiesterase 1, acid lysosomal | 250 | 326 |
| | SPTLC1 | Serine palmitoyltransferase, long chain base subunit 1 | 130 | 152 |
| | SULT2B1 | Sulfotransferase family, cytosolic, 2B, member 1 | 343 | 564 |
| | UGCG | UDP-glucose ceramide glucosyltransferase | 174 | 121 |
| **Desquamation** | KLK7 | Kallikrein-related peptidase 7 | 625 | 580 |
| | CASP14 | Caspase 14, apoptosis-related cysteine peptidase | 962 | 687 |
| **Antimicrobial peptide, innate immunity** | DEFB4 | Defensin, beta 4 | 629 | 10464 |

**Example 2**: Effect of an edelweiss extract on the gene expression of in-vitro reconstructed epidermis with and without UV irradiation:

### 1- Material and methods:

A) Test material and preparation: Edelweiss extract (ALPAFLOR^{®} EDELWEISS from DSM) at a concentration of 5 wt-% dry residue was prepared as a stock solution in 60% ethanol. The diluted edelweiss extract was topically applied on the epidermal models in 1.2 % ethanol water solution at 100 mg/cm2.
B) Biological model: Reconstructed human epidermis prepared as described in Poumay, Y., Dupont, F., Marcoux, S., Leclercq-Smekens, M., Hérin, M. & Coquette, A. (2004) A simple reconstructed human epidermis : preparation of the culture model and utilization in in-vitro studies. Arch. Dermatol. Res. 296, 203-211. Culture conditions: grown for 11 days at 37°C, 5% CO₂
C) Epidermis culture and treatment:
   The reconstructed human epidermises (RHE) were incubated in culture medium and then topically treated or not (for control) with edelweiss extract and then incubated for 24 hours.
   All experimental conditions were performed twice. After incubation RHE were irradiated or
   not with UVB+A) at 250 mJ/cm2. After irradiation the RHE were topically re-treated with the test extract and incubated for 6 or 24 hours. After the incubation the RHE were washed in phosphate buffered saline solution and immediately frozen at -80°C.
D) Analysis of Gene Expression by Quantitative PCR technology as PCR array:
   The expression of a selection of genes was analysed using RT-qPCR method on mRNA extracted from the RHE for each treatment. Before RNA extraction the replicas were pooled. The analysis of gene expression was then done in duplicates (n=2).
   Reverse transcription: Total RNA was extracted from each sample using TriPure solution reagent according to the supplier's advices. The amount and quality of RNA were evaluated using a lab-on-a-chip Bioanalyzer (Agilent Technologies). Potential contaminant traces of genomic DNA were removed using the DNAfree system (Ambion). The amount of the RNA was evaluated using Nanovue (GE Healthcare). The reverse-transcription of mRNA was conducted in presence of oligo(dT) and Superscript II reverse-transcriptase (Invitrogen). Nanovue was also used for quantification of cDNA and DNA concentration adjustment.

Quantitative PCR: The PCRs (Polymerase Chain Reactions) were performed using the Light Cycler system (Roche Molecular Systems Inc.) according to the supplier's instructions. This system allows rapid and powerful PCRs after determining analysis conditions of the test primers. The reaction mix contains diluted DNA, primers forward and reverse, reagent mix containing taq DNA polymerase, SYBR Green I and MgCl2. The incorporation of fluorescence in amplified DNA was continuously measured during the PCR cycles. This resulted in a fluorescence intensity versus PCR cycle plot allowing the evaluation of a relative expression (RE) value for each gene. The value selected for RE calculations is the output point (Ct) of the fluorescence curve. For the considered gene the higher the cycle number the lower is the mRNA quantity. The RE value was expressed in arbitrary units (AU) according to the formula: (1/2 number of cycles) x 106. For additional control we also quantified the expression level of three housekeeping genes namely Ribosomal protein S28, Glyceraldehyde-3-phosphate dehydrogenase and beta-Actin in both untreated control cells and treated cells. The mean relative expression level of the three housekeeping genes was set to 100% and the variation in expression level of individual three genes was within less than 100 ± 50%.

### 2- Results:

The edelweiss extract was able to rebalance key markers of the pro-inflammatory cascade like 5-Lipoxygenase, psoriasin (S100A7) and CXCL5, that was recently identified as key mediator of pain signalling provoked by UV irradiation as it is the case in sunburn situations.

Moreover, the edelweiss extract rebalanced UV induced immuno-suppression markers such as toll-like receptor 4 (TLR-4) that promotes photoimmunosuppression. *L. alpinum* extract inhibited TLR-4 expression and interleukin 4 receptor (IL4R) expression which is also a pro-inflammatory marker that stimulates interleukin 10 and is part of the UV induced immune-suppressive cascade.

All results are summarized in Table 2.

**Table 2: Gene expression results RHE after UV irradiation: Gene expression levels are shown in %. 100% level untreated control with UV irradiation**

| | 6 hours incubation after UV irradiation treated with Edelweiss Extract - 0.02% |
|---|---|
| **Gene name** | % Control (Mean HK) |
| | 100 |
| Ribosomal protein S28 | 84 |
| Glyceraldehyde-3-phosphate dehydrogenase | 120 |
| Actin, beta | 86 |
| S100 calcium binding protein A7 | 51 |
| Toll like receptor 4 | 53 |
| Interleukin 4 receptor | 19 |
| Chemokine(CXC motif) ligand 5 | 45 |
| Arachidonate 15-lipoxygenase | 43 |
| Arachidanate lipoxygenase 3 | 58 |
| Glutathione peroxidise 1 | 40 |
| Metallothionein 1G | 52 |

### Conclusion:

In summary the edelweiss extract up-regulated genes of key marker proteins responsible for the functionality of the cellular, lipid and antimicrobial skin barrier. Moreover, gene expression data indicated a reduced UV stress on epidermal cells. *L. alpinum* extract inhibited markers of oxidative stress, pro-inflammatory cascades, immuno-suppression and unpleasant sensations. These data altogether can be referred to several skin benefits such as skin barrier regeneration, skin barrier strengthening and skin protective properties.

## Claims

1. Use of a topical composition comprising an edelweiss extract for prevention of skin damages, and/or regeneration of the skin barrier function, and/or for strengthening of the skin barrier, and/or improvement of the natural skin protection functionality.

2. The use of a topical composition according to claim 1, wherein improvement of the natural skin protection functionality is **characterized by** induction of cellular differentiation markers, induction of genes contributing to barrier lipid synthesis, and induction of endogenous anti-microbial proteins.

3. The use of a topical composition according to any of the claims 1 or 2, wherein the topical composition comprises between 0.01 and 10 weight-% edelweiss extract.

4. The use of a topical composition according to any of the claims 1 or 2, wherein the topical composition comprises between 0.5 and 3 weight-% edelweiss extract.

5. The use of a topical composition according to any of the claims 1 to 4, wherein the topical composition is a cosmetic composition.

6. The use of a topical composition according to any of the claims 1 to 5, wherein the topical composition is a cream, an emulsion, a gel, an ointment, a lotion, a tincture, a spray, a mousse, a cleansing composition or foam.

7. The use of a topical composition according to any of the claims 1 to 6, wherein the topical composition further comprises at least one UV screening agent, and a conventional carrier.

8. A topical pharmaceutical composition comprising an edelweiss extract for use in the prevention and/or treatment of human skin barrier abnormalities.

9. A topical pharmaceutical composition according to claim 8, wherein the skin barrier abnormalities are selected from atopic dermatitis, allergic skin, psoriatic skin inflamed skin, abnormal skin re-epithelization, burned or sunburned skin, and wounded skin.

10. A method of regeneration of the skin barrier function and/or improvement of the natural skin protection mechanism which comprises topically administering an effective amount of an edelweiss extract to the appropriate skin area of a person in need of such treatment.

11. A method of preventing skin barrier abnormalities which comprises topically administering an effective amount of an edelweiss extract to the appropriate skin area of a person in need of such treatment.
